# EUROPEAN PATENT APPLICATION

(11) **EP 4 289 404 A1**
(43) Date of publication of application: **13.12.2023**
(21) Application number: 23178601.3
(22) Date of filing: 12.06.2023
(51) Int. Cl.: A61F 5/445

(54) **DISPOSABLE TUBE FOR USE DURING OSTOMY APPLIANCE ATTACHMENT**

(30) Priority: 10.06.2022 US 202217837672
(71) Applicant: Kelly, Mark A., Chesterfield, MO 63006 (US)
(72) Inventor: Kelly, Mark A., Chesterfield, MO 63006 (US)
(74) Representative: FRKelly

(57) **Abstract**

A disposable cupping tube for collecting urine from a stoma during change of a mounting plate of a two-piece ostomy appliance. The cupping tube formed of an elastomeric material with a first end adapted to fit over the stoma with a second end capped. A diaphragm with a check valve positioned between the first and second ends. When the cupping tube is squeezed and an end of the tube pressed against the peristoma skin an air seal is formed and the check valve opened with chambers of reduced pressure above and below the diaphragm.

## Description

### BACKGROUND OF THE INVENTION

### Field of the Invention

The present invention relates to a cupping tube for collection of urine during installation and replacement of a two-piece ostomy appliance.

### Brief Description of the Prior Art

Modern two-piece ostomy appliances include a mounting plate, commonly called a ring wafer or baseplate, and a collection pouch that is attached mechanically to the ring wafer or with an adhesive. Urine from a stoma drains into the collection pouch which may be emptied or changed a number of times whereas the ring wafer may last between 4 to 10 days before it needs to be replaced.

A problem for urostomy patients with the current systems is that during removal and replacement of the mounting plate, urine flows from the stoma and wets the patient's skin around the stoma. The urine can irritate the patient's skin, possibly start an infection in the stoma, and will interfere with the adhesive bonding of a replacement ring wafer. Replacing a ring wafer and making the area clean and dry is difficult especially for elderly people who are most likely to be a urostomy patient. There is also an insurance problem. If a ring wafer fails because it is not applied to dry skin, Medicare and other insurance carriers limit the number of ostomy appliances covered per period and ostomy appliances are expensive if they must be privately paid for.

### BRIEF SUMMARY OF THE INVENTION

In view of the above, it is an object of the present invention to provide a collection tube that facilitates installation and replcement a two-piece ostomy applicance by a patient or his or her care providers. Other objects and features of the invention will be in part apparent and in part pointed out hereinafter.

In accordance with the invention, a cupping tube for collecting fluid from a stoma is formed of a flexible elastomeric material with an internal space configured to accommodate the stoma at one end and closed at the other. When squeezed and pressed against the peristomal skin the cupping tube forms an air seal. A check valve is positioned between the first open and second closed ends dividing the cupping tube into an upper and a lower chamber of reduced air pressure when the cupping tube is squeezed and sealed against the peristomal skin. At the same time the check valve allows fluid from the stoma collected in the lower chamber to be drawn through the check valve into the upper chamber.

In some embodiments the cupping tube is formed of a silicone rubber and is closed with a removable plug. In other embodiments the inside diameter of the cupping tube is the same at the first and second ends such that plug will fit in either end. In a preferred embodiment, the outside diameter of the cupping tube is such that the a wafer ring may be slid along the sides of the cupping tube.

In another embodiment the diaphragm divides the cupping tube into an upper larger chamber and a smaller lower chamber, said upper larger chamber configured to receive the stoma and associated ureter catheters without mechanical interference.

The invention summarized above comprises the constructions hereinafter described, the scope of the invention being indicated by the subjoined claims.

### BRIEF DESCRIPTION OF THE SEVERAL VIEWS OF THE DRAWINGS

In the accompanying drawings, in which one of various possible embodiments of the invention is illustrated, corresponding reference characters refer to corresponding parts throughout the several views of the drawings in which:
Fig. 1 is a perspective view of a cupping tube in accordance with the present invention;

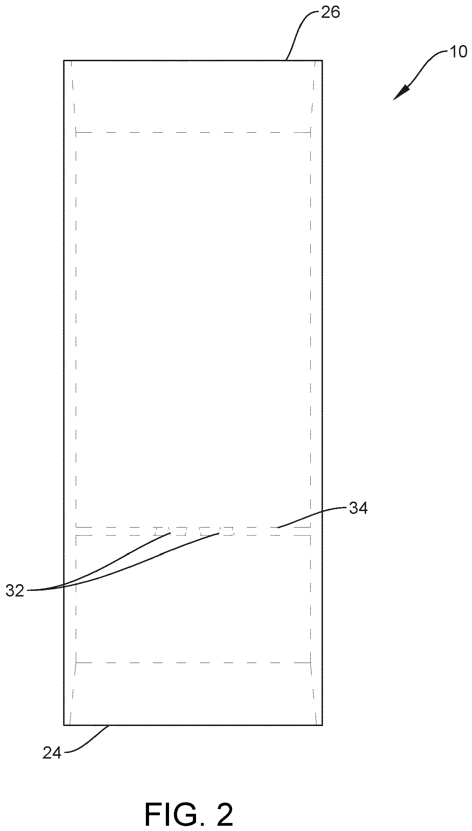

Fig. 2 is side elevation thereof;

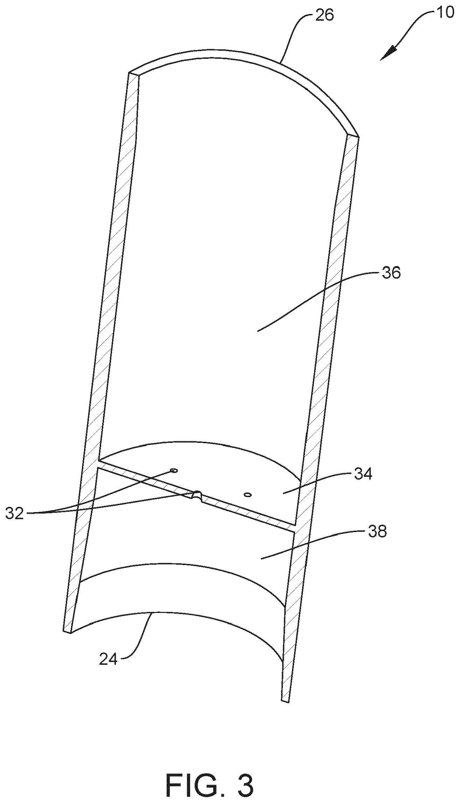

Fig. 3 is a sectional view taken along the plane of 2-2 in Fig. 1;
Fig. 4 shows anatomical details of an urostomy procedure;
Fig. 5 is a plan view of a stoma with extending ureter catheters;
Fig. 6 is a view of a patient with a stoma in his sidewall;
Fig. 7 is a view of the patient squeezing a cupping tube for attachment to his sidewall over the stoma;
Fig. 8 shows the cupping tube installed over the stoma;
Fig. 9 is a view of the patient peeling a backing tape from a ring wafer to reveal an adhesive layer;
Fig. 10 shows the patient sliding the ring wafer over the cupping tube;
Fig. 11 is a view of the patient making sure that the ring wafer has proper orientation;
Fig. 12 shows the patient pressing the ring wafer down to adhesively secure the wafer to his chest;
Fig. 13 shows the patient detaching the cupping tube from his chest which closes a check valve with any accumulated urine captured in the cupping tube;
Fig. 14 shows the cupping tube detached from the patient's chest; and,
Fig. 15 is a view showing the patient attaching a collection pouch to the ring wafer.

### DETAILED DESCRIPTION OF AT LEAST ONE PREFERRED EMBODIMENT OF THE INVENTION

Referring to the drawings more particularly by reference character, a cupping tube 10 for use during replacement of a ring wafer 12 in a two-piece ostomy appliance is formed of a flexible elastomeric material. Suitable elastomeric materials include, but are not limited to, silicone rubbers.

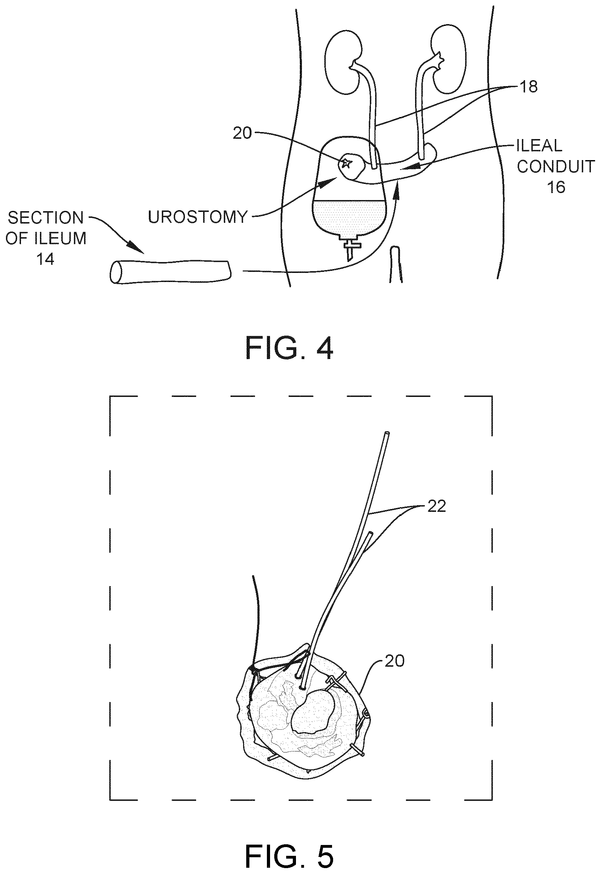

In urostomy surgery as shown in Fig. 4, a part of the small intestine (ileum 14) is cut and clamped at one end. This creates a new urine container (ileal conduit 16) or "bladder". The two ureters 18 that carry urine from the kidneys are attached to the new bladder while the open end of the new bladder is brought out through the abdominal to create a stoma 20 as shown in Fig. 5. During initial healing, catheters 22 connected to the ureters 18 pass out the stoma. If all goes to plan the catheters 22 are eventually removed.

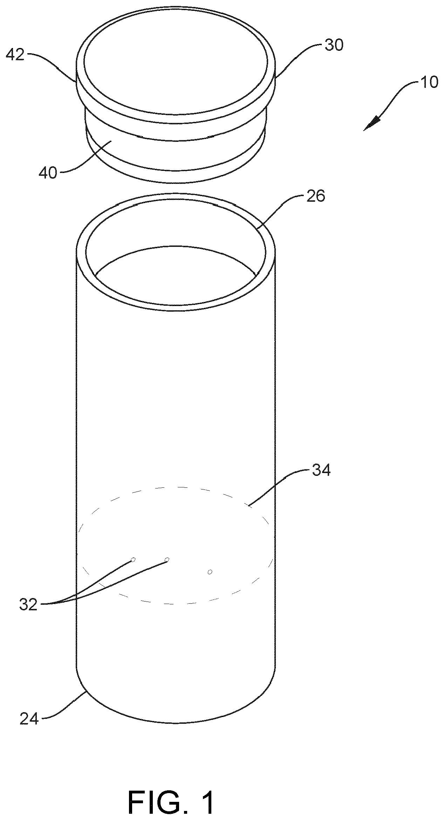

Cupping tube 10 includes a first end 24 and a second end 26. In the form illustrated in Fig. 1, first end 24 has an internal space configured to accommodate stoma 20 of a urostomy patient and be pressed against periostoma skin 28 at the base of stoma 20. Cupping tube 10 is closed at second end 26 which in the form illustrated is with a removable plug 30.

A check valve 32 in a diaphragm 34 is provided between first and second ends 24, 26 dividing cupping tube 10 into an upper larger chamber 36 and a lower smaller chamber 38. In one form, check valve 32 is a pinch valve.

When cupping tube is squeezed and first end 24 positioned over stoma and pressed against periostoma skin 28 an air seal is formed. Squeezing also opens normally closed pinch valve and when the squeeze is released upper and lower chambers 36, 38 are at reduced air pressure. Urine flowing out stoma 20 is initially captured in lower chamber 38 of cupping tube 10 and when the lower chamber is filled drawn up into upper chamber 36.

In the embodiment shown in the drawings, first and second ends 24, 26 have an inside diameter such that either end of cupping tube 10 may be positioned over stoma 20. This features makes cupping tube 10 suitable for use when catheters 22 are present which may be accommodated in upper larger chamber 36. When not required to accommodate catheters 22, use of cupping tube 10 other end down is preferred such that most of the urine is held by check valve 32 in the larger upper chamber when the cupping tube 10 is removed as described below. Catheters 22 are present during the hospital phase such that a patient and his or her caretakers may experience use of cupping tube 10 immediately after surgery while under medical supervision and then graduate to use of cupping tube 10 other end down when the catheters have been removed.

When first and second ends 24, 26 have the same inside diameter, plug 30 may be used with either end down. As shown in Fig. 1, plug 30 has a first portion 40 that fits within the end of cupping tube 10 for sealing the tube airtight. A second portion 42 of plug 30 has a larger diameter than first portion for capping off the end. First portion 40 has a length such that it fits deeply enough in cupping tube 10 to prevent seepage of fluid from stoma 20 out of the tube.

The cupping tube's utility as mentioned above is during the replacement of an adhesively attached ring wafer 12 of a two piece ostomy appliance. For which purpose, cupping tube 10 has an outside diameter such that wafer ring 12 may slide over cupping tube 10 during installation of a replacement wafer ring 12 and, optionally, also during removal of the ring wafer in need of replacement.

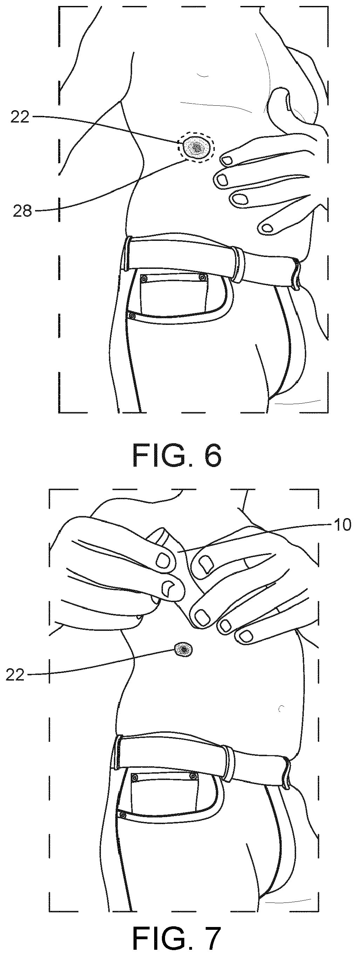

As shown in Figs. 6-15, in the replacement of a two-piece ostomy appliance, the first steps in the procedure are to remove the used pouch and then detach the adhesively attached ring wafer to which the pouch was attached. Stoma 20 of a urostomy patient has no sphincter such that urine will continuous ooze from the stoma. With the present system, cupping tube 10 is positioned over the stoma as soon as the pouch is removed and the existing ring wafer detached from the skin and slid over cupping tube 10. Alternatively, the spent ring wafer may be removed first and then cupping tube 10 attached as shown in Figs. 6-8.

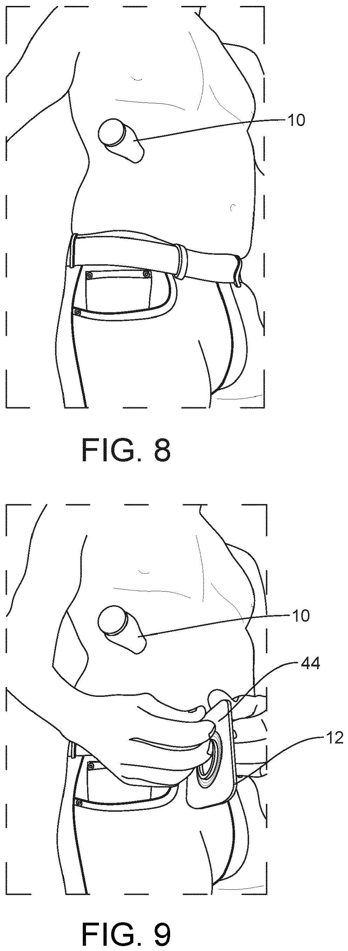

During attachment, cupping tube 10 is squeezed (Fig. 7) and the open end (first end or second end 24, 26 with the opposite end plugged 30) pressed against peristomal skin 28 (Fig. 8) to form an airtight seal. During initial healing, a user may have catheters 22 connected to ureters 18 as shown in Fig. 5 extending from the stoma. In which case, second end 26 of cupping tube 10 is passed over the stoma and catheters which are received in larger upper chamber 36. With either end of cupping tube 10 attached, urine seeping from stoma 20 is drawn into the upper and lower chambers 36, 38 through check valve 32.

Once the spent ring wafer is removed and cupping tube 10 attached, a user or his or her caretaker may take as much time as necessary to clean and prep the skin around the stoma. Without cupping tube 10, a pad is usually applied to the stoma in an attempt to soak up the urine. Application of a pad may mechanically injure the stoma and some urine is likely to escape the pad. This makes it very difficult to effectively clean the skin and prepare a dry skin surface necessary for attachment of replacement ring wafer 12. With cupping tube 10, prepping the skin is much easier.

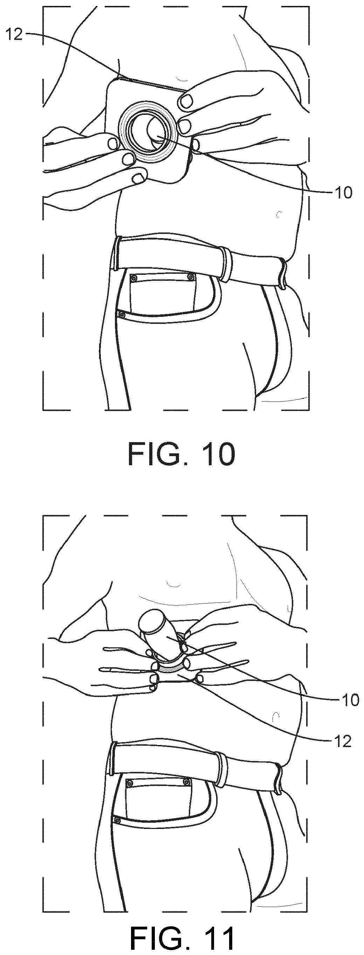

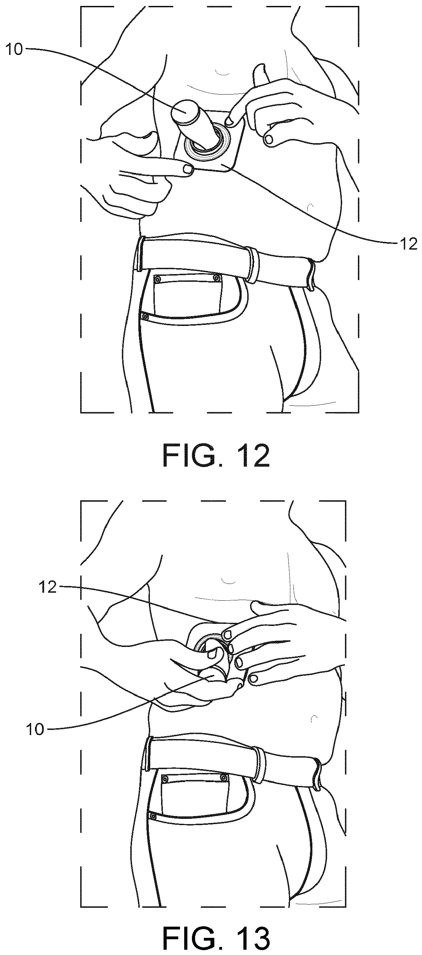

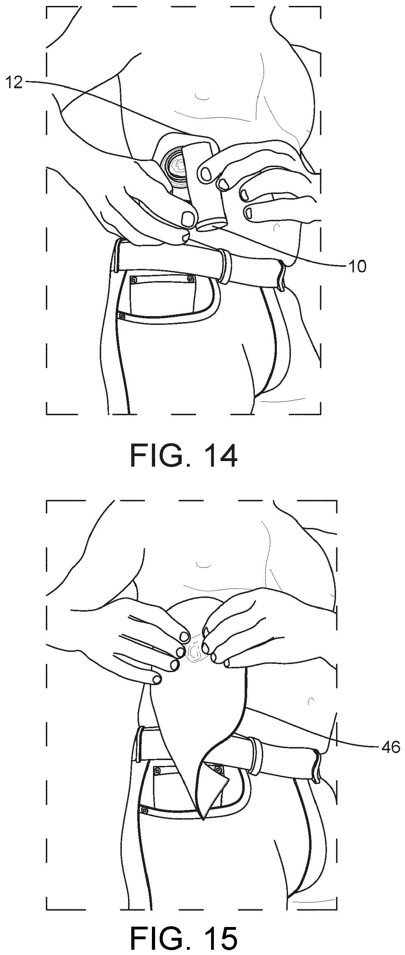

With applicant's system, a protective strip 44 may be removed from a replacement ring wafer 12 as shown in Fig. 9 revealing an adhesive layer on the underside of the wafer. Alternatively, the adhesive may be hand applied to the wafer or to the patient's skin before the ring wafer is slid over cupping tube 10 as shown in Fig. 10. Ring wafer 12 is then positioned and pressed against the user's skin adhesively sealing the wafer in place as shown in Fig. 11-12. During all which time, urine seeping from stoma 20 is drawn into cupping tube 10 and stoma is protected from mechanical injury. Cupping tube 10 may then removed as shown in Figs. 13-14 and a replacement pouch 46 secured to ring wafer 12 as shown in Fig. 15.

Urine is not considered a medically hazardous material. Hence cupping tube 10 filled with urine may be wrapped in a paper towel or the like and disposed of with ordinary trash. Placement of the replacement ring wafer 12 on clean, dry skin makes it less likely that the wafer will need to be changed earlier than the expected and insurance covered every 4 to 10 days of use. The procedure is also less traumatic for a patient making the change alone and for his or her caretaker if assisted.

In view of the above, it will be seen that the object of the invention is achieved and other advantageous results attained. As various changes could be made in the above constructions without departing from the scope of the invention, it is intended that all matter contained in the above description or shown in the accompanying drawings shall be interpreted as illustrative and not in a limiting sense.

## Claims

1. A cupping tube for collecting fluid from a stoma surrounded by peristomal skin, said cupping tube formed of a flexible elastomeric material with an internal space configured to accommodate the stoma, said cupping tube open at a first end forming an air seal against the peristomal skin when the cupping tube is squeezed and pressed against the peristomal skin, said cupping tube closed at a second end, said cupping tube having a check valve between the first and second ends dividing the cupping tube into an upper and a lower chamber of reduced air pressure when the cupping tube is squeezed and sealed against the peristomal skin, said check valve allowing fluid from the stoma collected in the lower chamber to be drawn through the check valve into the upper chamber.

2. The cupping tube of claim 1 wherein the check valve is a normally closed pinch valve which opens when the cupping tube is squeezed.

3. The cupping tube of claim 1 for use with a stoma resulting from urostomy.

4. The cupping tube of claim 1 wherein the elastomeric material is a silicone rubber.

5. A cupping tube for collecting fluid from a stoma projecting from the skin of a user during removal or installation of a wafer ring for an ostomy pouch, said stoma resulting from urostomy and surrounded by peristomal skin,
said cupping tube formed of a flexible elastomeric material and having first and second ends,
said first end of the cupping tube having an inside diameter such that the cupping tube may be positioned over a stoma and said second end closed with a removable plug,
said cupping tube has an outside diameter that is smaller than a diameter of the wafer ring so that the wafer ring may slide over the cupping tube,
said cupping tube forming an air seal against the peristomal skin when the cupping tube is squeezed and pressed against the peristomal skin,
said cupping tube having a diaphragm between the first and second ends dividing the cupping tube into an upper and a lower chamber of reduced air pressure when the cupping tube is squeezed and sealed against the peristomal skin, said diaphragm having at least one aperture for allowing fluid from the stoma collected in the lower chamber to be drawing through the aperture into the upper chamber.

6. The cupping tube of claim 5 wherein the at least one aperture includes a check valve.

7. The cupping tube of claim 6 wherein the check valve is a normally closed pinch valve which opens when the cupping tube is squeezed.

8. The cupping tube of claim 5 wherein the plug has a first portion that fits within the second end of the cupping tube for sealing the tube airtight, a second portion of the plug has a larger diameter than the first portion for capping off the second end of the cupping tube, said first portion of the plug has a length that it fits deeply enough in the cupping tube to prevent seepage of fluid from the stoma out of the tube.

9. A cupping tube for collecting fluid from a stoma projecting from the skin of a user during removal or installation of a wafer ring for an ostomy pouch, said stoma resulting from urostomy and surrounded by peristomal skin,
said cupping tube formed of a flexible elastomeric material and having first and second ends,
said first end and second ends of the cupping tube having an inside diameter such that the either end of the cupping tube may be positioned over a stoma with the other end closed with a removable plug,
said cupping tube has an outside diameter that is smaller than a diameter of the wafer ring so that the wafer ring may slide over the cupping tube,
said cupping tube forming an air seal against the peristomal skin when the cupping tube is squeezed and pressed against the peristomal skin,
said cupping tube having a diaphragm between the first and second ends dividing the cupping tube into a larger upper and a smaller lower chamber of reduced air pressure when the cupping tube is squeezed and sealed against the peristomal skin, said diaphragm having at least one aperture for allowing fluid from the stoma collected in the lower chamber to be drawing through the aperture into the upper chamber.

10. The cupping tube of claim 9 wherein the plug is on the first end and the second end is positioned over a stoma with catheters connected to the kidneys, said upper chamber being large enough to accommodation the stoma and the catheters.
